Europäisches Patentamt

European Patent Office

Office européen des brevets

(19)

(11) Publication number: **0 101 294**
**B1**

(12) **EUROPEAN PATENT SPECIFICATION**

(45) Date of publication of patent specification: **05.04.89**

(51) Int. Cl.⁴: **A 61 K 31/20,** A 61 K 9/00, A 61 K 47/00

(21) Application number: **83304607.1**

(22) Date of filing: **09.08.83**

(54) Composition for protecting and healing gastro-duodenal mucosa and the liver of mammals.

(30) Priority: **09.08.82 US 406558**
**21.07.83 US 515766**

(43) Date of publication of application:
**22.02.84 Bulletin 84/08**

(45) Publication of the grant of the patent:
**05.04.89 Bulletin 89/14**

(84) Designated Contracting States:
**AT BE CH DE FR GB IT LI LU NL SE**

(56) References cited:
**FR-A-2 231 379**
**US-A-3 792 179**

**CHEMICAL ABSTRACTS, Vol. 88, No. 21, May 22, 1978, p. 555, no. 152049c, Columbus, OH (US)**

(73) Proprietor: **THE REGENTS OF THE UNIVERSITY OF CALIFORNIA**
**2199 Addison Street**
**Berkeley California 94720 (US)**

(72) Inventor: **Hollander, Daniel**
**213 Via Dijon**
**Newport Beach, CA 92663 (US)**
Inventor: **Tarnawski, Andrzej Stanislaw**
**3108 Lincoln Way**
**Costa Mesa, CA 92626 (US)**

(74) Representative: **Burford, Anthony Frederick et al**
**W.H. Beck, Greener & Co. 7 Stone Buildings Lincoln's Inn**
**London WC2A 3SZ (GB)**

Courier Press, Leamington Spa, England.

**Description**

Ethanol, acetylsalicylic acid and taurocholic acid, a component of bile, may cause severe injury to the gastroduodenal mucosa of mammals, including experimental animals (e.g. rats) and humans, when these three agents separately or in conjunctions come into contact with the gastro-duodenal mucosa. The injuries to the gastro-duodenal mucosa caused by these three substances, which have been documented in various respected clinical journals, include the following: acute hemorrhagic gastritis with acute upper gastro-intestinal bleeding; gastric erosions and ulcer formation (particularly related to aspirin and taurocholic acid); and alkaline reflux syndrome occurring spontaneously or after gastric surgery.

Ethanol, in addition to causing injury to the gastro-duodenal mucosa, also produces severe injury to the liver in mammals, when ingested by said mammal including humans, as documented by elevation of certain serum enzymes and liver histology. Other hepatotoxic substances also produce liver damage.

Various prior art investigators have conducted a variety of experiments in an attempt to find a composition which will protect the gastro-duodenal mucosa and the liver from injury caused by ethanol and other toxic substances. It has been determined that various prostaglandins may prevent gastro-duodenal and liver injury produced by ethanol and other toxic substances. However, prostaglandins are expensive and have systemic side effects.

The Inventors have discovered that two relatively inexpensive fatty acids which are not only not harmful to the body but are necessary dietary substances will protect and/or heal the liver from injury induced by hepatotoxic substances and the gastro-duodenal mucosa from ethanol, acetylsalicylic acid and taurocholic acid induced injury. These two fatty acids are arachidonic acid and linoleic acid which are present, in relatively small amounts, in a normal diet. It is known that these two acids are precursors of prostaglandins. For example, previous *in vitro* studies have indicated that the gastric mucosa of various animals, including humans, is able to synthesize prostaglandins (including prostaglandin $E_2$ and $F_2$) from arachidonic acid when the stomach lining is ground up (including the mucosa) and the arachidonic acid mixed therewith. It is also known that arachidonic acid is converted to prostaglandin $E_2$ in the small intestine. However, when either arachidonic or linoleic acid are taken orally, either per se or in a food, they are not able to be absorbed by the gastro-duodenal mucosa and the arachidonic acid and linoleic acid which is absorbed by the small intestine distal to the duodenum and converted to prostaglandin cannot reach the gastro-duodenal mucosa because they are metabolized and deactivated by the lungs.

FR—A—2231371 (corresponding to GB—A—1476624) discloses the intra-peritoneal use of arachidonic acid and its esters in the treatment of anxiety and excitation. Optionally, linoleic acid may also be present in the arachidonic acid or ester-containing medicament. Polyoxyethylenated oleic glyceride is disclosed as a suitable carrier for methyl arachidonate.

US—A—3792179 discloses (in Example 12) a tranquilizing lozenge obtained by compressing a mixture of micronized arachidonic acid, powdered polyethylene glycol 400 and powdered polyethylene glycol 600.

The Inventors have found that if arachidonic and/or linoleic acid are absorbed distal to the duodenum and two fatty acids have absolutely no effect on protecting and healing the gastro-duodenal mucosa or the liver.

The present invention is based on the surprising discovery that the oral administration, to mammals, of arachidonic and linoleic acids will protect and/or heal the liver from ethanol and other hepatotoxic substances and the gastro-duodenal mucosa from injury induced by ethanol, acethylsalicylic acid and taurocholic acid, if the arachidonic acid or linoleic acid is first mixed with a pharmaceutically acceptable water solubilizing compound which makes the fatty acid soluble in water and allows the fatty acids to be absorbed by the gastro-duodenal mucosa. The Inventors have found that when the fatty acid is absorbed by the mucosa, prostaglandins (e.g. prostaglandin $E_2$) are synthesized. They believe that this is what protects and/or heals the liver and the gastro-duodenal mucosa.

The water solubilizing compounds, which are also pharmaceutically acceptable, may be a variety of compounds providing that they will solubilize either of the fatty acids in an aqueous medium, such as the aqueous fluids secreted by the gastric and duodenal mucosa. The preferred water solubilizing compounds of the present invention are the non-ionic surface active agents such as ethylene oxide surface active agents; including polyethenoxy ethers of alkyl phenols, polyethenoxy ethers of alcohols, and polyethenoxy ethers of mercaptans; difunctional and polyfunctional polyethenoxy ethers (e.g. condensation of ethylene oxide with bis-phenols), polyethenoxy esters, particularly of tall oil acids as well as rosins and alkylated benzoic acid or polyethenoxy compounds with amide links.

The concentration of the linoleic or arachidonic acid in the water solubilizing compound is not particularly important but we have found that, in general, the concentration will be between about 10 and 200 mM, and, preferably, the concentration is between 30 and 150 mM, preferably between 60 and 120 mM, per ml water-solubilizing compound (calculated as Poloxamer 188).

The dosages of the fatty acids of the present invention which are necessary to achieve the desired results can be determined by those skilled in the art and, in general, a dosage of at least about 5 mg/kg will be used. There is no theoretical limit on the maximum amount although excellent results will be obtained using 75 or 100 mg/kg and therefore there is no reason to use higher dosages. The presently preferred dosages are between about 15 or 18 mg/kg and about 25 mr 30 mg/kg.

The Inventors have also found that the fatty acids of the present invention may be administered many hours before the gastro-duodenal mucosa is contacted by one or more of the three potentially harmful agents and still protect the gastro-duodenal mucosa. The Inventors believe, for example, that the instant composition may be administered up to 3 hours prior to ingestion of any of the toxic substances and still protect the gastro-duodenal mucosa and the liver.

The Inventors have observed that in order to protect the liver and/or the gastro-duodenal mucosa it is necessary that the gastric mucosa absorb either the arachidonic acid or the linoleic acid, or mixtures of both. Either the liver or the jejunal portion of the small intestine will not protect the gastro-duodenal mucosa from the harmful effect of the three agents.

In order to more fully understood the present invention reference will be made to the following examples, which are for the purposes of illustration only.

In the following examples the water solubilizing compound was a non-ionic surfactant prepared by the addition of ethylene oxide to polypropylene glycol which is marketed under the trademark Pluronic F-68 (sometimes hereinafter abbreviated as PL). PL's CTFA name is Poloxamer 188. PL is a member of a family of block copolymers manufactured under the trademark Pluronic Polyols. PL contains 80 wt.% ethylene oxide and has a molecular weight of 8,350. In the examples or studies PL was used as a 5 mM solution in a saline solution of 0.9% sodium chloride adjusted to a pH of 8.0 with sodium hydroxide.

The arachidonic acid was a grade 1 acid which was stored in the dark at minus 20°C in sealed ampules until used. When the ampule was opened the arachidonic acid was transferred immediately to a vial which was under a stream of nitrogen and contained the 5 mM PL saline solution. The mixture was stirred vigorously and the pH adjusted to 8.0 with sodium hydroxide. The resulting solution became optically clear and remained so for 24 hours. Arachidonic acid solutions of 30, 60 and 120 mM in 5 mM PL were prepared immediately prior to their administration to the animals.

The linoleic acid used in the subsequent examples was also solubilized in the 5 mM PL saline solution as indicated above.

The animals used in the examples were Spraque-Dawley rats (225 to 250 grams bodyweight). Twenty-four hours prior to the experimentation the animals were fasted in wire-bottomed cages to prevent coprophagy.

Study 1: Arachidonic acid mucosal protection for 3 hours after ethanol administration

Animals were given by gavage 1 ml of either saline, or 5 mM PL solution in saline, or 1 ml of the PL-saline solution containing arachidonic acid in 30, 60 and 120 mM concentrations respectively. One-half hour or 1 hour later, 2 ml of absolute ethanol were instilled into the stomach by gavage. Three hours later the animals were sacrificed and their stomachs removed.

Study 2: Arachidonic acid mucosal protection for 15 hours after ethanol administration

One ml of 120 mM arachidonic acid solution (37 mg arachidonic acid) or 5 mM Pl solution was instilled into the stomach 1 hour prior to the intragastric administration of 2 ml ethanol. The animals were killed 15 hours after ethanol administration and their stomachs were examined.

Study 3: Intragastric versus intrajejunal administration of arachidonic acid

The next series of experiments was designed to compare the effect of intragastric to intrajejunal pretreatment with arachidonic acid on ethanol-induced gastric mucosal injury. The animals were anesthetized with nembutal (50 mg nembutal per kg body weight intraperitoneally), their abdomens were opened and either 1 ml of 120 mM arachidonic acid or 5 mM PL was installed intragastrically or 1 ml of 120 mM arachidonic acid intrajejunally with a polyethylene catheter introduced through a small duodenal incision. The pylorus was ligated immediately after solution instillation. One hour later the gastric contents were removed and 2 ml of ethanol were instilled through the catheter. The stomach was examined 2 hours later.

Computerized analysis of mucosal changes

A camera with 100 mm macro lens was used to photograph the mucosal surface of the stomach. The same type of film and identical conditions of specimen distance from the lens, light intensity and film development were used throughout the study. Color slides were evaluated by a computerized image analysis. Each slide was converted to a black-and-white video image consisting of 65,000 points. White represented normal gastric mucosa, while gray or black represented hemorrhagic changes. The value of each point was measured on a digital scale which was based on color intensity and had a range of 0 to 255. Absolute white was given a 255 value while absolute black was given a value of 0. Boundaries delineating the glandular mucosa were drawn on the digital video image with a sonic pen. All spatial and contrast information was stored on a magnetic disk. Each glandular mucosal area was classified interactively by slicing the spectrum of mucosal values into 2 or more subgroups and by displaying the resultant simplified digital image for verification with the more complex analog photographic input. Once a classification scheme was established, a spatial rendition of the picture's point was printed and plotted to scale on paper. A summary of the computer results was also printed and included boundary values for each gray-level slice and the absolute and relative areas of each class. The areas of hemorrhagic changes were expressed as the percentage of the entire gastric glandular mucosal area.

Histological studies

Mucosal specimens 5 mm in width were cut from the forestomach to the pylorus. The specimens were obtained (a) 60 minutes after intragastric administration of 1 ml of solution containing either saline, PL or 120 mM arachidonic acid or (b) 3 hours after intragastric instillation of 2 ml of ethanol in animals which had received 1 ml of solution containing either saline or PL or 120 mM arachidonic acid 1 hour before ethanol. Specimens were fixed in buffered 10% formalin and stained. Coded specimens were examined "blindly" by light microscopy. In the histological evaluation special attention was paid to the continuity of the surface epithelium layer, presence or absence of hemorrhagic erosions, edema of the submucosa and infiltration with polymorphonuclear neutrophils.

Ultrastructural studies by scanning electron microscopy

Gastric mucosal specmens, fixed in 3.5% buffered glutaraldehyde were processed routinely for scanning electronmicroscopy and evaluated in a JOEL-35 scanning electronmicroscope operated at 20 kV.

Prosaglandin E$_2$ (PGE$_2$) measurements

PGE$_2$ was measured by direct radioimmunoassay using antisera generated in rabbits by immunization of PGE$_2$ linked to bovine thyroglobulin. For additional confirmation of PGE$_2$ measurements, samples were also subjected to bioassay. After addition of approximately 5000 CPM of $^3$H-labeled PGE$_2$ for recovery calculations, samples were adjusted to pH 4.3 with 0.5 M phosphate buffer and extracted twice with ethyl acetate. The organic layer was subjected to thin layer chromatography (benzene:dioxane:acetic acid, 90:60:3), and the PGE$_2$ fraction was extracted with methanol, dried and reconstituted in 0.9% NaCl for assay. A bioassay cascade (according to Vane) was used to assay simultaneously the samples in 2 aliquot sizes and on 3 rat stomach strips. The concentration of PGE$_2$ in the gastric contents were measured in 2 groups of rats 30 or 60 minutes following intragastric installation of 1 ml of 120 mM arachidonic acid or 1 ml PL in order to determine the extent of arachidonic acid conversion to PGE$_2$ by the gastric mucosa. The spontaneous release of PGE$_2$ into the gastric lumen was assessed in animals receiving PL pretreatment only. In addition, PGE$_2$ concentration in the original 120 mM arachidonic acid solution was measured before intragastric instillation.

Results

Study 1

Pretreatment of animals with saline followed by ethanol administration resulted in severe gastric hemorrhagic lesions. Pretreatment with the PL solubilizer similarly failed to prevent severe mucosal hemorrhagic changes (Table I). In contrast, intragastric pretreatment of rats with 1 ml of 30, 60 and 120 mM arachidonic acid 1 hour prior to the administration of 2 ml of ethanol reduced the development of gastric hemorrhagic lesions in a dose dependent manner (Table I). Pretreatment with 1 ml of 120 mM arachidonic acid either 30 or 60 minutes prior to ethanol instillation protected the gastric mucosa against ethanol injury (Table I).

Study 2

The gastric mucosa of animals which had been pretreated with PL only and 1 hour later had been given 2 ml of ethanol, demonstrated very advanced hemorrhagic changes 15 hours later (Table I). The hemorrhagic changes were seen throughout the fundus, and to a lesser degree in the antrum, and consisted of dark red elongated bands. In contrast, the gastric mucosa of animals which had been pretreated with intragastric instillation of 1 ml of 120 mM arachidonic acid solution 1 hour prior to the instillation of ethanol appeared normal 15 hours later.

Study 3

After the pylorus had been ligated, arachidonic acid was instilled intragastrically or intrajejunally in order to determine whether its protection was a result of local or systemic effects. Intragastric pretreatment with 1 ml of 120 mM arachidonic acid offered significant protection of the gastric mucosa against the development of ethanol-induced hemorrhagic lesions (Table I). In contrast, intrajejunal administration of arachidonic acid to animals with ligated pylorus did not protect the gastric mucosa against injury produced by intragastric administration of ethanol (Table I).

Histological changes

Intragastric administration of 1 ml of saline or 5 mM PL did not produce any histological changes. Arachidonic acid instillation into the stomach did not change the appearance of the gastric mucosa but did increase the discharge of mucous granules from the surface epithelial cells.

Histologic changes in gastric mucosa 3 hours after ethanol in groups pretreated with PL or arachidonic acid (1 ml of 120 mM) are shown in Table II. Three hours after intragastric instillation of 2 ml of ethanol to the animals receiving saline or PL pretreatment, the fundic mucosa showed presence of large areas of mucosal necrosis extending 2/3 to 3/4 of the mucosal thickness. These changes occurring in all animals were also present in the glandular mucosa adjacent to the squamous epithelial mucosa of the forestomach, and were characterized by severe congestion of the lamina propria, severe edema of the submucosa, and

4

accumulation of leukocytes in the submucosa and basal lamina propria. Large areas of mucosa were devoid of surface epithelial cells. Sections of macroscopically uninvolved mucosa showed diffuse edema of the submucosa and mucus discharge from the surface epithelial cells.

Mucosal specimens from the stomachs of animals pretreated with arachidonic acid were normal 3 hours after ethanol instillation showing intact surface epithelium with only a few areas of superficial disruption. Some oedema of the submucosa and some accumulation of leukocytes were detected. The layer of mucous granules of the surface epithelial cells was thinner and displayed occasional focal defects.

Scanning electronmicroscopy results

Rats pretreated with PL showed severe disruption of the mucosal surface and craters of completely denuded lamina propria, at 3 hours after ethanol. In contrast in arachidonic acid pretreated rats 3 hours after ethanol mucosal surface was nondisrupted and normal appearance.

$PGE_2$ determination

The concentrations of $PGE_2$ in gastric content 30 and 60 minutes after intragastric instillation of arachidonic acid or PL are shown in Table III. The values of $PGE_2$ in the gastric content of those animals that received intragastric instillation of arachidonic acid were 5,000—13,000 higher than the values seen in those animals that received intragastric instillation of PL only. The latter values represent the rate of spontaneous release of $PGE_2$ into the gastric luman. $PGE_2$ was present in the initial arachidonic acid solution (prior to intragastric instillation) in amount equivalent to 0.0027% of the arachidonic acid concentration.

Table I shows the results of the computerized analysis of gastric hemorrhagic areas in respective groups. The results of hemorrhagic mucosal areas are expressed as percent of total gastric glandular mucosal area. In all studies, except group E, ethanol (ETOH) was instilled intragastrically 1 hour after 0.9% NaCl, PL or arachidonic acid (AA). In Group E, ethanol (ETOH) was instilled 30 minutes after arachidonic acid.

TABLE I

| Experiments | Number of animals | Number of hours after ethanol when stomach evaluated | Hemorrhagic area*(%) |
|---|---|---|---|
| Study 1 | | | |
| A) 0.9% NaCl | 4 | 3 | 37.0 |
| B) PL | 10 | 3 | 33.8 |
| C) 30 mM AA+PL | 5 | 3 | 15.4 |
| D) 60 mM AA+PL | 4 | 3 | 3.0 |
| E) 120 mM AA+PL | 4 | 3 | 0.8 |
| F) 120 mM AA+PL | 10 | 3 | 0.6 |
| Study 2 | | | |
| G) PL | 7 | 15 | 27.4 |
| H) 120 mM AA+PL | 7 | 15 | 0.6 |
| Study 3 | | | |
| K) 120 mM AA intra-jejunally+PL | 5 | 2 | 28.1 |
| L) 120 mM AA intra-gastrically+PL | 4 | 2 | 5.3 |
| M) PL intragastrically | 6 | 2 | 32.2 |

*Percent of total glandular mucosal area

TABLE II

Histological changes in gastric mucosa 3 hours after ETOH instillation (2 ml absolute ethanol)

| Group pretreated with | The surface epithelium layer | Hemorrhagic erosions | Edema of submucosa accumulation of polymorphonuclears |
|---|---|---|---|
| PL (1 ml) | Disrupted, large area of mucosal surface denuded | Present, multiple penetrating to basal mucosa | Present |
| AA (1 ml 120 mM+PL | Present, continuous (minor focal defects seen occasionally) | Absent | Present |

TABLE III

Prostaglandin $E_2$ concentration in gastric contents after intragastric instillation 1 ml PL or AA (1 ml, 120 mM)

| Groups | $PGE_2$ concentration (ng/ml) | |
|---|---|---|
| | 30 min | 60 min |
| PL | 0.55±0.14 | 0.88 |
| AA | 7360.0* | 4100.0* |

*$p < 0.01$

Study 4: Linoleic acid mucosal protection

The rats were given intragastric treatment by peroral tube 1 ml of PL solution; 1 ml of the PL solution containing 120 mM linoleic acid (LLC) or 240 mM LLC; or 1 ml of 240 mM oleic acid solubilized in 5 mM PL. One hour after ethanol instillation animals were sacrificed, the stomach was opened along the greater curvature, rinsed with 0.9% NaCl, examined and scored visually and photographed. Macroscopic and histologic mucosal changes were evaluated as in the studies with arachidonic acid.

Results:

Rats receiving intragastric pretreatment with PL solution only had 3 hours after ethanol macroscopic hemorrhagic lesions involving 35.8±2% of glandular mucosal area. Treatment with oleic acid did not change significantly occurrence of mucosal hemorrhagic lesions −29.1±3%. Rats receiving pretreatment with 120 mM and 240 mM LLC had significantly decreased macroscropic hemorrhagic lesions −6.5±1% and 1.3±0.5% respectively (p<0.01) 3 hours after ethanol. Macroscopic protection rat gastric mucosa against ethanol was similar to that afforded by arachidonic acid except for the presence of marked edema in the forestomach (containing squamous, paraepidermoidal epithelium) 3 hours after ethanol in group pretreated with linoleic acid (C).

Mucosal Histology:

In rats receiving PL or oleic acid pretreatment 3 hours after ethanol deep hemorrhagic necrotic lesions penetrating into muscularis mucosa were present and the continuity of the surface epithelium was disrupted. Three hours after ethanol in animals pretreated with linoleic acid no deep necrotic lesions were present and the surface epithelium was mostly restored with flat or cuboidal immature cells. In the forestomach edema of submucosa was present however.

Study 5: Arachidonic acid mucosal protection against taurocholic acid

Rats were given intragastric treatment by peroral tube with 1 ml of PL solution or 1 ml of PL solution containing 120 mM arachidonic acid (AA). The rats then received, intragastrically, 3 mls of a 80 mM solution of taurocholic acid (TCA) dissolved in 0.1 n in HCl, adminstered twice: one or two hours after PL or AA pretreatment. Animals were sacrificed 3 hours after second TCA administration.

Results:

Gastric mucosa was assessed macroscopically and histologically for damage in a similar manner as in studies with ethanol. In PL pretreated animals 3 hours after second dose of TCA gastric mucusa showed

6

macroscopically presence of linear hemorrhagic lesions occupying 7.5±0.5% of total mucosal area. These lesions corresponding histologically to necrotic lesions were similar to those seen after ethanol. In AA pretreated animals mucosal lesions were not detectable macroscopically and histologically at 3 hours after second TCA dose, proving protective effect of AA against TCA induced gastric mucosal necrosis.

Study 6: Arachidonic and linoleic acid mucosal protection against acetylsalicylic acid

Rats were given intragastric treatment by peroral tube with 1 ml of PL solution, 1 ml of PL solution containing 120 mM arachidonic acid (AA), or 1 ml of PL solution containing 240 mM linoleic acid (LLC). One hour after such treatment a suspension of 200 mg/kg bodyweight of aspirin in 0.1 n HCl containing 1% methyl cellulose was administered intragastrically in 1 ml volume. The animals were sacrified 4 hours after acetyl salicylic acid administration.

Studies:

1) Macroscopic quantitation of mucosal changes based on scoring system estimating presence or absence of hemorrhagic lesions, their number and severity graded on arbitrary scale 1 to 4. Lesion index, expressed as an average score (number of lesions×severity) was estimated per each group of animals.
2) Mucosal histology
3) Scanning electron microscopy

Results:

Macroscopic mucosal changes at 4 hours after acetyl salicylic acid were 80±6 in the PL group and significantly less (2±0.3 and 8±1) in AA and LLC groups respectively. Histologic evaluation 4 hours after aspirin showed presence of deep necrotic lesions penetrating into midportion of the mucosa in PL pretreated rats. The continuity of the surface epithelium in regions between necrotic lesions was extensively disrupted. In contrast in AA and LLC groups at 4 hours after aspirin deep necrotic lesions did not occur and mucosal surface epithelium was continuous. The scanning electromicroscopy revealed the same findings extensive mucosal damage after aspirin in PL pretreated animals had nearly normal mucosa in AA and LLC pretreated rats. These studies showed that AA and LLC acid are very effective in protecting rat gastric mucosa against acetylsalicylic acid induced gastric mucosal necrosis.

The compositions of our invention are also effective in healing gastric-duodenal ulceration because of the production of $PGE_2$ which is known to heal such ulceration.

Study 7:

40 rats were given, by gavage, either 1 ml of 5 mM PL solution (as a control) or a 1 ml of 120 mM arachidonic acid in the 5 mM PL solution. One hour later the rats were given 2.5 ml of 100% ethanol and the animals sacrificed 24 hours later.

The liver was then removed and specimens fixed for 2 hours in 3.5% purified glutaraldehyde (buffered in 0.1 M phosphate of 7.4). The liver specimens were washed with buffer and postfixed in 1% osmium tetroxide at 0°C for 1 hour. After dehydration with graded ethanol the tissue was embedded in epoxy resin. Silver gray thin sections of the specimen were double stained with 2% uranyl acetate followed by lead citrate and examined with an RCA electron microscope.

Results:

The following ultrastructural changes occurred in the hepatocytes of control rats receiving PL solution only followed by 2.5 ml of 100% ethanol 1) focal cytoplasmic necrosis, 2) swelling and irregularity of most of the mitochondria with distortion of the size and shape of the cristae, with intramitochondiral dense granules and occasional myelin figures present, 3) prominent proliferation and dilatation of the endoplasmic reticulum, 4) presence of lipid vacules in the cytoplasm and 5) glycoden depletion and chromatin replacement of most of the nuclear area. These morphological changes were present in all the animals studied. In rats receiving AA pretreatment followed by ethanol, most of the alcohol induced changes were virtually prevented except for lipid vaculization which was significantly reduced.

It is apparent from this example, that a single dose of ethanol administered orally to rats produces prominent ultrastructural changes in the hepatocytes. These changes include alteration of mitochrondria and of cytoplasmic reticulum as well as focal cytoplasmic necrosis (degradation). These changes are similar to those seen in human liver after acute dose alcohol administration.

The present example demonstrated further that oral pretreatment with a single dose of only 37 mg of AA protects the liver against acute injury induced with ethanol.

When the compositions of the invention are administered in unit dosage form, each unit dose may contain, for example, 125 mg of 2 g, usually 250 mg to 1 g.

**Claims for the Contracting States BE CH DE FR GB IT LI LU NL SE:**

1. The use of a fatty acid selected from arachidonic acid, linoleic acid and mixtures thereof for the manufacture of an orally administrable medicament for the treatment of mammalian gastro-duodenal mucosa and/or mammalian liver, said medicament containing also a pharmaceutically acceptable water-solubilizing compound for said fatty acid.

2. The use of a composition consisting essentially of (a) a fatty acid selected from arachidonic acid, linoleic acid and mixtures thereof and (b) a pharmaceutically acceptable water-solubilizing compound for said fatty acid in the manufacture of an orally administrable medicament for the treatment of mammalian gastro-duodenal mucosa and/or mammalian liver.

3. A use as claimed in Claim 1 or Claim 2, wherein the said water-solubilizing compound is a surface active agent.

4. A use as claimed in Claim 3, wherein the said surface active agent is non-ionic.

5. A use as claimed in Claim 4, wherein said surface active agent is a polyethenoxy surface active agent.

6. A use as claimed in Claim 5, wherein said surface active agent is a polyethenoxy ether of a glycol.

7. A use as claimed in Claim 6, wherein said surface active agent is Poloxamer 188.

8. A use as claimed in any one of the preceding Claims, wherein the fatty acid concentration in the medicament is 10 to 200 mM per ml water-solubilizing compound (calculated as Poloxamer 188).

9. A use as claimed in Claim 8, wherein the said concentration is 30 to 150 mM.

10. A use as claimed in Claim 9, wherein the said concentration is 60 to 120 mM.

11. A use as claimed in any one of the preceding Claims wherein the medicament is in unit dosage form containing 250 mg to 1 g of said fatty acid per unit dose.

12. A use as claimed in any one of the preceding Claims wherein the medicament consists essentially of said fatty acid and said water-solubilizing compound.

13. A use as claimed in any one of the preceding Claims, wherein the medicament is for protecting and/or healing mammalian liver from injury induced by a hepatotoxic substance.

14. A use as claimed in Claim 13, wherein the hepatotoxic substance is ethanol.

15. A use as claimed in any one of the preceding Claims, wherein the medicament is for protecting and/or healing mammalian gastro-duodenal mucosa from injury induced by ethanol, acetylsalicylic acid and/or taurocholic acid.

16. An orally administrable pharmaceutical composition for mammalian intragastric treatment, said composition comprising a fatty acid selected from arachidonic acid, linoleic acid and mixtures thereof and a pharmaceutically acceptable polyoxyethylene - polyoxypropylene block copolymer in an amount sufficient to water-solubilize said fatty acid.

17. A composition as claimed in Claim 16, wherein said copolymer is Polyoxamer 188.

18. A composition as claimed in Claim 16 or Claim 17, wherein the fatty acid concentration is 10 to 200 mM per ml water-solubilizing compound (calculated as Poloxamer 188).

19. A composition as claimed in Claim 18, wherein the said concentration is 30 to 150 mM.

20. A composition as claimed in Claim 19, wherein the said concentration is 60 to 120 mM.

21. A composition as claimed in any one of Claims 16 to 20 in unit dosage form containing 250 mg to 1 g of said fatty acid per unit dose.

22. A composition as claimed in any one of Claims 16 to 21 consisting essentially of said fatty acid and said copolymer.

**Claims for the Contracting State AT:**

1. A method of preparing an orally administrable pharmaceutical composition for mammalian intragastric treatment, said method comprising mixing a fatty acid selected from arachidonic acid, linoleic acid and mixtures thereof and a pharmaceutically acceptable polyoxyethylene-polyoxypropylene block copolymer in an amount sufficient to water-solubilize said fatty acid.

2. A method as claimed in Claim 1, wherein said copolymer is Poloxamer 188.

3. A method as claimed in Claim 1 or Claim 2, wherein the fatty acid concentration is 10 to 200 mM per ml water-solubilizing compound (calculated as Poloxamer 188).

4. A method as claimed in Claim 3, wherein the said concentration is 30 to 150 mM.

5. A method as claimed in Claim 4, wherein the said concentration is 60 to 120 mM.

6. A method as claimed in any one of Claims 1 to 5 in unit dosage form containing 250 mg to 1 g of said fatty acid per unit dose.

7. A method as claimed in any one of Claims 1 to 6 consisting essentially of said fatty acid and said copolymer.

**Patentansprüche für die Vertragsstaaten BE CH DE FR GB IT LI LU NL SE:**

1. Verwendung einer Fettsäure, ausgewählt aus Arachidonsäure, Linolsäure und Mischungen davon zur Herstellung eines oral verabreichbaren Medikaments zur Behandlung von Säugetier-Gastro-Duodenal-Mucosa und/oder Säugetier-Leber, wobei das Medikament auch eine pharmazeutisch annehmbare wasserlöslich machende Verbindung für die Fettsäure enthält.

2. Verwendung einer Zusammensetzung, die im wesentlichen aus

a) einer Fettsäure, ausgewählt aus Arachidonsäure, Linolsäure und Mischungen davon, und

EP 0 101 294 B1

b) einer pharmazeutisch annehmbaren wasserlöslich-machenden Verbindung für die Fettsäure besteht, zur Herstellung eines oral verabreichbaren Medikaments zur Behandlung von Säugetier-Gastro-Duodenal-Mucosa und/oder Säugetier-Leber.

3. Verwendung nach Anspruch 1 oder 2, wobei die wasserlöslich machende Verbindung ein oberflächenaktives Mittel ist.

4. Verfahren nach Anspruch 3, wobei das oberflächenaktive Mittel nicht ionisch ist.

5. Verwendung nach Anspruch 4, wobei das oberflächenaktive Mittel ein Polyethenoxy oberflächenaktives Mittel ist.

6. Verwendung nach Anspruch 5, wobei das oberflächenkative Mittel ein Polyethenoxyether eines Glykols ist.

7. Verwendung nach Anspruch 6, worin das oberflächenaktive Mittel Poloxamer 188 ist.

8. Verwendung nach einem der vorhergehenden Ansprüche, wobei die Fettsäurekonzentration in dem Arzneimittel 10 bis 200 mM pro ml wasserlöslichmachender Verbindung (berechnet als Poloxamer 188) ist.

9. Verwendung nach Anspruch 8, wobei die Konzentration 30 bis 150 mM ist.

10. Verwendung nach Anspruch 9, wobei die Konzentration 60 bis 120 mM ist.

11. Verwendung nach einem der vorhergehenden Anspruch, wobei das Arzneimittel in einer Einheits-Dosierungsform mit 250 mg bis 1 g der Fettsäure pro Einheits-Dosis vorliegt.

12. Verwendung nach einem der vorhergehenden Ansprüche, wobei das Medikament im wesentlichen aus der Fettsäure und der wasserlöslich-machenden Verbindung besteht.

13. Verwendung nach einem der vorhergehenden Ansprüche, wobei das Medikament zum Schutz und/oder zur Heilung von Säugetier-Leber von Schädigung, hervorgerufen durch eine hepatotoxische Substanz, verwendet wird.

14. Verwendung nach Anspruch 13, wobei die hepatotoxische Substanz Ethanol ist.

15. Verwendung nach einem der vorhergehenden Ansprüche, wobei das Arzneimittel zum Schutz und/oder zur Heilung von Säugetier-Gastro-Duodenal-Mucosa von Schädigung, hervorgerufen durch Ethanol, Acetylsalicylsäure und/oder Taurocholsäure, verwendet wird.

16. Oral verabreichbare pharmazeutische Zusammensetzung zur intragastrischen Behandlungvon Säugetieren, wobei die Zusammensetzung eine Fettsäure, ausgewählt aus Arachidonsäure, Linolsäure und Mischungen davon, und ein pharmazeutisch annehmbares Polyoxyethylen-Polyoxypropylen - Blockcopolymer in einer Menge, die zum Wasserlöslichmachen der Fettsäure ausreicht, enthält.

17. Zusammensetzung nach Anspruch 16, wobei das Copolymer Poloxamer 188 ist.

18. Zusammensetzung nach Anspruch 16 oder 17, wobei die Fettsäurekonzentration 10 bis 200 mM pro ml wasserlöslichmachender Verbindung (berechnet als Poloxamer 188) beträgt.

19. Zusammensetzung nach Anspruch 18, wobei die Konzentration 30 bis 150 mM beträgt.

20. Zusammensetzung nach Anspruch 19, wobei die Konzentration 60 bis 120 mM beträgt.

21. Zusammensetzung nach einem der Ansprüche 16 bis 20, in einer Einheits-Dosierungs-Form, die 250 mg bis 1 g der Fettsäure pro Einheits-Dosis enthält.

22. Zusammensetzung nach einem der Ansprüche 16 bis 21, die im wesentlichen aus der Fettsäure und dem Copolymer besteht.

**Patentansprüche für den Vertragsstaat AT:**

1. Verfahren zur Herstellung einer oral verabreichten pharmazeutischen Zusammensetzung zur intragastrischen Behandlung von Säugetieren, wobei das Verfahren das Mischen eines Fettsäure, ausgewählt aus Arachidonsäure, Linolsäure und Mischungen davon, und einem pharmazeutischen annehmbaren Polyoxyethylen - Polyoxypropylenblockcopolymer in einer Menge, die ausreichend zum Wasserlöslichmachen der Fettsäure ist, umfaßt.

2. Verfahren nach Anspruch 1, wobei das Copolymer Poloxamer 188 ist.

3. Verfahren nach Anspruch 1 oder 2, wobei die Konzentration der Fettsäure 10 bis 200 mM pro ml wasserlöslichmachender Verbindung (berechnet als Poloxamer 188) beträgt.

4. Verfahren nach Anspruch 3, wobei die Konzentration 30 bis 150 mM beträgt.

5. Verfahren nach Anspruch 4, wobei die Konzentration 60 bis 120 mM beträgt.

6. Verfahren nach einem der Ansprüche 1 bis 5 in Einheits - Dosierungs-Form mit 250 mg bis 1 g der Fettsäure pro Einheits-Dosis.

7. Verfahren nach einem der Ansprüche 1 bis 6 bestehend im wesentlichen aus der Fettsäure und dem Copolymer.

**Revendications pour les Etats Contractants BE CH DE FR GB IT LI LU NL SE:**

1. Utilisation d'un acide gras choisi entre l'acide arachidonique, l'acide linoléïque et leurs mélanges pour la fabrication d'un médicament administrable par voie orale pour le traitement des muqueuses gastro-duodénales chez un mammifère et/ou du foie chez un mammifère, ledit médicament contenant également un composé de solubilisation dans l'eau, acceptable sur le plan pharmaceutique, pour ledit acide gras.

2. Utilisation d'une composition consistant essentiellement en a) un acide gras choisi entre l'acide

9

arachidonique, l'acide linoléïque et leurs mélanges, et b) un composé de solubilisation dans l'eau, acceptable sur le plan pharmaceutique, pour ledit acide gras, dans la fabrication d'un médicament administrable par voie orale pour le traitement des muqueuses gastro-duodénales chez un mammifère et/ou du foie chez un mammifère.

3. Utilisation selon l'une ou l'autre des revendications 1 et 2, dans laquelle ledit composé de solubilisation dans l'eau est un agent tensio-actif.

4. Utilisation selon la revendication 3, dans laquelle ledit agent tensio-actif est non ionique.

5. Utilisation selon la revendication 4, dans laquelle ledit agent tensio-actif est un agent tensio-actif polyéthènoxy.

6. Utilisation selon la revendication 5, dans laquelle ledit agent tensio-actif est un polyéthènoxyéther d'un glycol.

7. Utilisation selon la revendication 6, dans laquelle ledit agent tensio-actif est le Poloxamer 188.

8. Utilisation selon l'une quelconque des revendications précédentes, dans laquelle la concentration en acide gras dans le médicament est de 10 à 200 mM par ml de composé de solubilisation dans l'eau (calculé en Poloxamer 188).

9. Utilisation selon la revendication 8, dans laquelle ladite concentration est de 30 à 150 mM.

10. Utilisation selon la revendication 9, dans laquelle ladite concentration est de 60 à 120 mM.

11. Utilisation selon l'une quelconque des revendication précédentes, dans laquelle le médicament est sous forme posologique unitaire contenant de 250 mg à 1 g dudit acide gras par dose unitaire.

12. Utilisation selon l'une quelconque des revendications précédentes, dans laquelle ledit médicament consiste essentiellement en ledit acide gras et ledit composé de solubilisation dans l'eau.

13. Utilisation selon l'une quelconque des revendications précédentes, dans laquelle le médicament est destiné à protéger et/ou à soigner le foie d'un mammifère de lésions provoquées par une substance hépatotoxique.

14. Utilisation selon la revendication 13, dans laquelle la substance hépatotoxique est l'éthanol.

15. Utilisation selon l'une quelconque des revendications précédentes dans laquelle le médicament est destiné à protéger et/ou à soigner les muqueuses gastro-intestinales d'un mammifère de lésions provoquées par l'éthanol, l'acide acétylsalicylique et/ou l'acide taurocholique.

16. Composition pharmaceutique administrable par voie orale pour le traitement intragastrique d'un mammifère, ladite composition comprenant un acide gras choisi entre l'acide arachidonique, l'acide linoléique et leurs mélanges, et un copolymère séquencé polyoxyéthylène - polyoxypropylène acceptable sur le plan pharmaceutique, en quantité suffisante pour solubiliser dans l'eau ledit acide gras.

17. Composition selon la revendication 16, dans laquelle ledit copolymère est le Poloxamer 188.

18. Composition selon la revendication 16 ou la revendication 17, dans laquelle la concentration en acide gras est 10 à 200 mM par ml de composé de solubilisation dans l'eau (calculé en Poloxamer 188).

19. Composition selon la revendication 18, dans laquelle ladite concentration est 30 à 150 mM.

20. Composition selon la revendication 19, dans laquelle ladite concentration est 60 à 120 mM.

21. Composition selon l'une quelconque des revendications 16 à 20, sous forme posologique unitaire contenant 250 mg à 1 g dudit acide gras par dose unitaire.

22. Composition selon l'une quelconque des revendications 16 à 21, consistant essentiellement en ledit acide gras et ledit copolymère.

**Revendications pour l'Etat Contractant AT:**

1. Procédé de préparation d'une composition pharmaceutique administrable par voie orale pour le traitement intragastrique chez un mammifère, ledit procédé consistant à mélanger un acide gras choisi entre l'acide arachidonique, l'acide linoléïque et leurs mélanges, et un copolymère séquencé polyoxyéthylène - polyoxypropylène acceptable sur le plan pharmaceutique, en quantité suffisante pour solubiliser dans l'eau ledit acide gras.

2. Procédé selon la revendication 1, dans lequel ledit copolymère est le Poloxamer 188.

3. Procédé selon la revendication 1 ou 2, dans lequel la concentration en acide gras est 10 à 200 mM par ml de composé de solubilisation dans l'eau (calculé en Poloxamer 188).

4. Procédé selon la revendication 3, dans lequel ladite concentration est 30 à 150 mM.

5. Procédé selon la revendication 4, dans lequel ladite concentration est 60 à 120 mM.

6. Procédé selon l'une quelconque des revendications 1 à 5, dans lequel la composition est sous forme posologique unitaire contenant de 250 mg à 1 g dudit acide gras par dose unitaire.

7. Procédé selon l'une quelconque des revendications 1 à 6, dans lequel la composition consiste essentiellement en ledit acide gras et ledit copolymère.